# EUROPÄISCHE PATENTSCHRIFT

(11) **EP 1 145 727 B1**
(45) Veröffentlichungstag und Bekanntmachung des Hinweises auf die Patenterteilung: **14.06.2006**
(21) Anmeldenummer: 01109136.0
(22) Anmeldetag: 12.04.2001
(51) Int. Cl.: A61M 5/44, A61M 1/00, A61M 39/12

(54) **Pumpe zum sterilen Fördern erwärmter Tumeszenzlösung**
Pump for sterile feeding of heated tumescence solution
Pompe pour la délivrance sous condition stérile de solution tumescente chauffée

(30) Priorität: 12.04.2000 DE 10017953; 28.11.2000 WO PCT/DE00/04232
(43) Veröffentlichungstag der Anmeldung: 17.10.2001
(73) Patentinhaber: Möller Medical GmbH & Co.KG, 36043 Fulda (DE)
(72) Erfinder: Sattler, Gerhard, 64297 Darmstadt (DE)
(74) Vertreter: Hebing, Norbert

(56) Entgegenhaltungen:
- FR-A- 2 753 103
- RU-C- 2 039 575
- US-A- 4 798 590
- US-A- 5 178 606
- US-A- 5 244 463
- US-A- 5 395 320
- US-A- 5 447 493
- US-A- 6 039 048

## Beschreibung

### Technisches Gebiet

Die Erfindung betrifft eine Tumeszenzpumpe zum sterilen Fördern erwärmter Tumeszenzlösung in das Gewebe eines Patienten, insbesondere zur Regionalanästhesie des intrakutanen und subkutanen Gewebes.

### Stand der Technik

In dem Dokument US-A-6,039,048 ist eine Vorrichtung zur Liposuktion unter Zuhilfenahme einer Ultraschallquelle offenbart. Hierbei wird mittels einer handelsüblichen Infusionspumpe die Flüssigkeit in das Gewebe eingebracht.

Das Dokument US-A-5,447,493 zeigt eine Vorrichtung zur Liposuktion, bei der mittels einer Rollenpumpe die Tumeszenzlösung zugeführt und gleichzeitig durch Umkehrung der Pumprichtung das Fett aus dem Körperinneren abgesaugt werden kann.

Das Dokument US-A-5,395,320 zeigt noch eine computergesteuerte Infusionspumpe, welche in den Parametern Durchfluss, Gesamtmenge und Zeit einstellbar ist. Die Steuerung erfolgt hier über einen Mikrokontroller. Die Messung des Durchflusses erfolgt mittels eines Tropfenzählers 32.

Die Tumeszenz-Lokalanästhesie (TLA) ist von dem Dermatologen und Pharmakologen Jeffrey A. Klein, assoziierter Professor an der Universitäts-Hautklinik in Irvine, Kalifornien, entwickelt und in der Januarausgabe 1987 des "Journal of Cosmetic Surgery" beschrieben worden. Diese Methode der lokalen Betäubung ist ursprünglich zur Erleichterung der Absaugung von subkutanem Fettgewebe aus kosmetischer Indikation gedacht gewesen.

Geschichtlich gesehen ist die Grundvoraussetzung zur Anwendung der Liposuktion die Bereitstellung einer "Einrichtung", mit der das in den Anfängen dieser Technik noch nicht vorbehandelte Fettgewebe mit Hohlnadeln abgesaugt werden kann. Der Eingriff in der anfänglich ausgeführten Form ist aufgrund starker Blutverluste und Risiken durch die Narkose nicht ungefährlich.

Eine weitere wichtige Voraussetzung zur erfolgreichen Anwendung dieser Methode ist die Bereitstellung einer geeigneten Lösung, die vor dem Absaugen eingebracht wird und so das Absaugen des Fettgewebes leichter durchführbar gestaltet. Neben der Verwendung neuer Sauger ist die sog. "wet technique" von Gerard Illouz, Paris eingeführt worden. Dabei wird mit Hyaluronidase vermischte Kochsalzlösung vor dem Absaugen in das Fettgewebe eingespritzt.

Auf der Grundlage dieser "wet technique" und unter dem berufspolitischem Druck, sich als operativ tätiger Dermatologe auf ambulante, in lokaler Betäubung durchführbare Eingriffe zu beschränken, entwickelte dann der Dermatologe und Pharmakologe Jeffrey A. Klein die Tumeszenz-Lokalanästhesie.

Die Weiterentwicklung der Tumeszenz-Lokalanästhesie zeichnet sich durch den Einsatz immer feinerer Saugkanülen aus. Auch das Einsatzgebiet der Technik der Tumeszenz-Lokalanästhesie ist in der jüngsten Vergangenheit erweitert worden. Parallel zur Entwicklung der Saugkanülen und Saugtechnik ist auch die Einspritztechnik weiterentwickelt worden.

Zur geschichtlichen Entwicklung und zur Beschreibung des allgemeinen Standes der Technik auf diesem Fachgebiet wird auf das Fachbuch "Tumeszenz-Lokalanästhesie - Praktische Anwendung", B. Sommer, G. Sattler, C.W. Hanke (Hrsg.), erschienen 1999 im "Springer-Verlag Berlin Heidelberg" verwiesen, auf das - wie im übrigen auch auf allen anderen in dieser Anmeldung genannten Stand der Technik - zur Erläuterung aller hier nicht im einzelnen beschriebenen Fachbegriffe und Einzelheiten ausdrücklich Bezug genommen wird.

Im Abschnitt 9 dieses Fachbuchs sind auch verschiedene Pumpspritzsysteme, überwiegend mechanische Pumpspritzen, verschiedener Hersteller beschrieben.

Bei der Durchführung von Liposuktionen, bei denen nach neuesten Erkenntnissen häufig bis zu ca. 6 l entsprechend verdünnter Lösung über einen längeren Zeitraum und unter Gewährleistung einer möglichst konstanten Flüssigkeitstemperatur injiziert werden müssen, sind auch elektrisch betriebene "automatische" Systeme im Einsatz. Bei den derzeit im Einsatz befindlichen Pumpen handelt es sich vorwiegend um mechanische Rollenpumpen bzw. Laborpumpen. Diese Pumpen besitzen den Nachteil, das sie für den genannten Einsatzzweck zu ungenau sind. Die bei nach neuesten Erkenntnissen durchzuführenden Infiltrationen können bei Einbringung größerer Mengen TLA-Lösung mehr als 1 Stunde dauern. Insbesondere dann, wenn mit Verteilersystemen für die zu injizierende Lösung gearbeitet wird, erweisen sich die derzeit im Einsatz befindlichen Pumpen als zu ungenau. Als besonders wichtig haben sich die Einhaltung der vorberechneten Menge an TLA-Lösung, die optimale Verteilung der Lösung im Gewebe, die richtige, nicht zu hohe Infiltrationsgeschwindigkeit, ein nicht zu hoher Infiltrationsdruck und die Gewährleistung einer konstanten Temperatur der Infiltrationsflüssigkeit herausgestellt.

Diese Forderungen werden mit den aus dem Stand der Technik bekannten Tumuleszenzsystemen nicht erfüllt. Vielmehr handelt es bei den zur Zeit im Einsatz befindlichen Pumpen um gewöhnliche Rollen- bzw. Laborpumpen, bei denen die in einer bestimmten Zeiteinheit zu injizierende Flüssigkeitsmenge durch einfaches Ein- und Ausschalten geregelt wird.

Die bisher im Einsatz befindlichen Pumpen erfüllen auch nicht die Anforderungen hinsichtlich der Beständigkeit gegenüber Reinigungs- und Desinfektionsmitteln.

Ein weiteres häufig auftretendes Problem ist die Gewährleistung der Dichtheit der Schlauchverbindungen, insbesondere an den Verbindungsstellen (Kupplungen) der Schlauchverbindungen. Bei erhöhtem Druck, wie er insbesondere beim unbeabsichtigten Abklemmen der Verbindungsschläuche auftreten kann, halten die bisher eingesetzten Verbindungen diesem erhöhten Druck nicht stand.

Weiterhin hat es sich für den behandelnden Arzt als sehr bedeutsam herausgestellt, alle relevanten Daten, wie z.B. Zeit, Druck, Temperatur und Durchflussmenge, während des Injektionsvorganges zu erfassen und zu registrieren.

Außerhalb des Fachgebietes Tumeszenzpumpen sind die verschiedensten medizinischen Pumpen bekannt. Hierzu wird auf die DE 31 38 478 C2, die DE 35 87 826 T2, die DE 39 12 405 C1, die DE 40 02 061 A1, die DE 195 03 350 C1, die US-PS4 598 697, die US-PS 5 716 194 oder die US-PS 5 853 387 verwiesen. Alle aus diesen Druckschriften bekannten Pumpsysteme sind - unabhängig davon, dass es sich diesen Pumpsystemen zumeist nicht um Tumeszenzpumpen handelt - nicht geeignet, die vorstehend angegebenen Eigenschaften in ihrer Gesamtheit zu erfüllen.

Insbesondere ist keines der bekannten Pumpsysteme in der Lage, mit einem Einmal- bzw. Einweg-Schlauchbesteck ohne Verwendung eines Durchflußsensors eine konstante Flussrate und/oder einstellbare Gesamtmenge der einzubringenden Tumeszenzlösung zu gewährleisten.

### Darstellung der Erfindung

Aufgabe der Erfindung ist es, eine Tumeszenzpumpe zum sterilen Fördern erwärmter Tumeszenzlösung in das Gewebe eines Patienten anzugeben, die die Einhaltung der vorberechneten Menge an TLA-Lösung, die optimale Verteilung der Lösung im Gewebe, die richtige, nicht zu hohe Infiltrationsgeschwindigkeit, einen nicht zu hohen Infiltrationsdruck und die Beibehaltung einer konstanten Temperatur der Infiltrationsflüssigkeit während der gesamten Zeitdauer der Infiltration gewährleistet, ohne dass die in einem Beutel oder dgl. bevorratete Tumeszenzlösung mit anderen Teilen als einem Einmal- bzw. Einweg-Schlauchbesteck in Berührung kommt.

Ferner soll die Pumpe selbsttätig dafür sorgen, dass zu hohe Drücke im Schlauchsystem bzw. -besteck zum Patienten hin nicht auftreten können. Ein unbeabsichtigtes Lösen der Schlauchverbindungen soll sicher vermieden werden. Die Pumpe muss gewährleisten, dass alle für die Behandlung wichtigen Daten während des gesamten Infiltrationsvorgangs erfasst und wiederabrufbar gespeichert werden. Die Pumpe soll ferner beständig gegenüber Reinigungs- und Desinfektionsmitteln sein.

Eine erfindungsgemäße Lösung dieser Aufgabe ist im Patentanspruch 1 angegeben. Weiterbildungen der Erfindung sind Gegenstand der abhängigen Ansprüche.

Erfindungsgemäß wird die Aufgabe dadurch gelöst, dass eine Tumeszenzpumpe zum sterilen Fördern erwärmter Tumeszenzlösung in das Gewebe eines Patienten verwendet wird, die in an sich bekannter Weise als Schlauchpumpe ausgebildet ist. Um mit einer Schlauchpumpe bzw. Schlauch-Rollenpumpe die (vorgewählte) Gesamtmenge der einzubringenden vorgewärmten Tumeszenzlösung und die (vorgewählte) Flussrate (zeitliche Einbringungsmenge) ohne Verwendung eines Durchflusssensors exakt zu steuern, d.h. nicht zu regeln, wird ein steriles Einmal-Schlauchset verwendet, dessen Strömungswiderstand und dessen Volumen innerhalb enger Grenzen bekannt und insbesondere konstant ist, so dass die von der Pumpe pro Zeiteinheit geförderte Flüssigkeitsmenge auch die Menge ist, die in das Gewebe des Patienten eingebracht wird. Anders ausgedrückt, erzeugt das Schlauchbesteck weder "momentane Quellen bzw. Senken" für die geförderte Flüssigkeit.

Die Steuereinheit steuert insbesondere die Drehzahl der Pumpe bevorzugt auf einen konstanten Wert und beendet nach dem Erreichen von Vorgabewerten den Pumpvorgang automatisch. Die Genauigkeit der Pumpe wird insbesondere dadurch gewährleistet, dass die (elektronische) Steuereinheit während einer Umdrehung der beispielsweise mit zwei Antriebsrollen ausgestatteten Pumpe die Umdrehungsgeschwindigkeit der Pumpe genau auf die geförderte Menge an Flüssigkeit einstellt. Anhand der Umdrehungszahl ist dann eine genaue Bestimmung der geförderten Durchflussmenge und nach Integration über die Förderzeit der gesamten geförderten Flüssigkeitsmenge möglich.

Eine besondere Bedeutung besitzt in diesem Zusammenhang das Material des Pumpschlauches, das eine hohe Flexibilität bei gleichzeitiger Druckbeständigkeit aufweist. Erst durch den Einsatz dieses Materials ist einen genaue Abstimmung der Elektronik auf die exakte Durchflussmenge pro "Hub" möglich. Gleichzeitig sind medizinische Anforderungen hinsichtlich der Sterilität zu erfüllen. Erfindungsgemäß wird ein spezielles Kunststoffmaterial eingesetzt, dass eine Festigkeit von 55 Shore ⁺/- 5 Shore aufweist.

Die Forderung hinsichtlich der Druckbegrenzung wird zum einen dadurch gelöst, dass die Antriebsrollen federgelagert sind. Die Federn sind auf Druck beanspruchbar, wobei der notwendige Druck so gewählt wird, dass die Federn bei einer ungewollten Blockade des Schlauchsytems, beispielsweise bei einem Druck von 2,5 Bar im Schlauchsystem, nachgeben. Wenn ein solcher Druck im System auftritt, geben die Federn nach, die Antriebsrollen können den Pumpschlauch nicht mehr zusammendrücken und es kann kein weiterer Druckaufbau erfolgen.

Die Steuereinheit bzw. Elektronik der Pumpe gewährleistet weiter, dass die Durchflussmenge an Flüssigkeit während einer definierten Zeiteinheit variabel einstellbar ist. Weiterhin ist es möglich, neben dem Parameter der konstanten Durchflussmenge auch die Gesamtmenge an Flüssigkeit, die injiziert werden soll, einzustellen. Nach Erreichen der vorbestimmten Menge schaltet die Pumpe automatisch ab. Ein Signal, z.B. ein akustisches oder optisches Signal, zeigt die Beendigung des Pumpvorganges an.

Die Pumpe ist mit mindestens einer Ausgabeeinheit bzw. Schnittstelle für Heizschalen ausgestattet. Über diese Schnittstelle ist(sind) die Heizschale(n) mit der Pumpe verbunden. Die Heizschalen werden über die Schnittstelle an der Pumpe mit Energie versorgt. Gleichzeitig erfasst ein Wärmesensor die Temperatur der vorgewärmten Beutel mit Tumeszenzlösung an der Heizschale. Mittels des erfassten Signals erfolgt die Regelung der Heizung, um die Beutel mit Tumeszenzlösung auf einer vorgegebenen Temperatur zu halten.

Die Tumeszenzpumpe ist mit einem auswechselbaren sterilen Schlauch-Set ausgerüstet, welches auf die Förderleistung der Pumpe derart abgestimmt ist, dass die erforderliche hohe Genauigkeit, der zeitlichen Einbringmenge an Flüssigkeit bei Vorgabe des Flüssigkeitsdrucks gewährleistet ist und dass gleichzeitig eine kontaminationsfreie Förderung der Tumeszenzlösung gesichert ist. Dabei hat der Pumpschlauch eine besondere Bedeutung. Neben einer erforderlichen sehr hohen Maßhaltigkeit des Schlauches, zeichnet er sich durch die oben bereits erwähnten Eigenschaften aus, die im Zusammenhang mit der Einstellung der Fördergenauigkeit bereits beschrieben wurden.

Das Schlauchsystem besteht wbevorzugt aus dem Ansaugschlauch, der mit dem Einstechdorn verbunden ist, und dem Verbindungsschlauch zum Injektionsinstrument oder zum Verteiler.

Die Verbindungen der Schläuche zur Pumpe, untereinander bzw. mit dem Verteiler oder dem Einstechdorn wird mittels bekannter Luer-Verbindungen realisiert. Die feste Verbindung dieser Luer-Verbindungen mit dem Schlauch bereitete in der Vergangenheit bei erhöhtem Druck im System Probleme, da die Verbindung des Verbindungsstückes zum Schlauch hin häufig undicht wurde. Zur Vermeidung dieses Problems wird erfindungsgemäß ein Schlauchanschluss eingesetzt, der an seinem zum Schlauch weisenden Ende, das dem Innendurchmesser des Schlauches angepasst ist, eine konische Erweiterung aufweist. Das Schlauchende wird über diesen Konus hinweg gezogen und unlösbar verbunden, vorzugsweise mittels Spezialkleber verklebt. Über den Schlauch wird danach außen eine Hülse geschoben, die wiederum dem Außendurchmesser des Schlauches straff angepasst ist. Diese Hülse wird ebenfalls unlösbar mit dem Schlauch verbunden. Eine solche unlösbare Verbindung lässt sich natürlich auch zwischen 2 Schläuchen herstellen.

Die Tumeszenzpumpe weist eine Ausgabeeinheit bzw. Schnittstelle auf, über die alle variablen Pumpdaten, wie z.B. Zeit, Druck, Temperatur und Durchflussmenge, während des Pumpvorgangs erfasst und gespeichert werden können. Während oder nach Beendigung des Pumpvorgangs sind diese Daten abrufbar. Dies kann über einen angeschlossenen Drucker oder über einen Computer erfolgen.

Das Pumpengehäuse ist bevorzugt aus einem speziellen Kunststoff gefertigt, der gegenüber allen in der Medizintechnik gebräuchlich verwendeten Reinigungs- und Desinfektionsmitteln beständig ist. Selbstverständlich ist das Pumpengehäuse auch gegen die Tumeszenzlösung beständig. Vorzugsweise wird der Kunststoff "PET-Novadron" verwendet.

In einer weiteren vorteilhaften Ausgestaltung ist die Tumeszenzpumpe mit einem Fußschalter ausgestattet, mit dem der Pumpvorgang in Gang gesetzt, unterbrochen bzw. beendet werden kann.

In einer besonders vorteilhaften Ausgestaltung wird die Tumeszenzpumpe mittels einer Befestigungseinrichtung mit einem Chirurgieabsauger verbunden. Das hat den Vorteil, dass beide für die Behandlung notwendigen technischen Geräte an einem Platz aufstellbar sind, wodurch die Übersichtlichkeit und Ordnung im Behandlungsraum erhöht wird. Ein weiterer wesentlicher Vorteil für den behandelnden Arzt besteht darin, dass über einen Doppelfußschalter beide Geräte bedienbar sind, ohne dass der Blick vom Patienten zur Betätigung der Tumeszenzpumpe oder des Chirurgieabsaugers weg gerichtet werden muss.

### Kurze Beschreibung der Zeichnungen

Die Erfindung wird nachfolgend anhand von Ausführungsbeispielen unter Bezugnahme auf die Zeichnung beschrieben, in der zeigen:
- Fig. 1: ein bevorzugtes Ausführungsbeispiel einer erfindungsgemäßen Tumeszenzpumpe mit Bedienoberfläche,
- Fig. 2: das Schlauchsystem,
- Fig. 3: eine Anordnung mit Heizschale und eingelegtem Kochsalzlösungsbeutel, Tumeszenzpumpe und Schlauchsystem mit Verteiler,
- Fig. 4: eine Heizschale für Flüssigkeitsbeutel,
- Fig. 5: eine Schlauchverbindung mit Luer-Verbindung, und
- Fig. 6: eine Schlauchverbindung zwischen zwei Schläuchen.

### Darstellung von Ausführungsbeispielen

Zunächst wird die Funktionsweise der Tumeszenzpumpe beschrieben:

Ein bevorzugt vorgewärmter Beutel mit Kochsalzlösung wird in eine der Temperatur-geregelten Heizschalen eingehängt. Die regelbare Heizung der Heizschale dient dazu, den Kochsalzbeutel warm zu halten. Es können insbesondere zwei Heizschalen an der Tumeszenzpumpe angeschlossen werden, die den Beutel praktisch vollsätndig umgeben. Die geregelte Flächenheizung der Heizschale wird mit der Ansteuerelektronik bzw. der Steuereinheit beispielsweise über eine 6-polige Schnittstelle (Anschlußkabel) mit der Pumpe verbunden. Über diese Schnittstelle ist auch der Temperatursensor mit der in der Pumpe befindlichen Temperaturregelung verbunden. Mit dem steril verpackten Schlauchsystem wird eine kontaminationsfreie Förderung der Kochsalzlösung erreicht. Das Schlauchset wird mit dem Pumpschlauchsegment in die Schlauchpumpe eingehängt.

Das eigentliche Pumpschlauchsegment (Außendurchmesser bevorzugt 8mm) befindet sich zwischen den Adaptionsstücken, welche in die Schlauchhalterung der Pumpe eingehängt werden. Mit dem Pumpschlauchsegment fest verbunden ist der beispielsweise 1,5 m lange PVC-Ansaugschlauch mit Einstechdorn für die Entnahme der Kochsalzlösung aus dem Vorratsbeutel.

Der Verbindungsschlauch dient zur Förderung der Kochsalzlösung aus der Schlauchpumpe bis zum Verteiler. Der Verbindungsschlauch wird mit der Luer-Verbindung einmal am Pumpenausgang und einmal am Verteiler angeschlossen.

Mit dem grünen Ein- und Ausschalter auf der linken Pumpenseite wird die Pumpe in Betriebsbereitschaft gebracht.

Die Betriebsbereitschaft wird durch eine grüne LED angezeigt. Sollte beim Einschalten der Pumpe die rote LED gleichzeitig leuchten, so wird signalisiert, dass die Frontscheibe der Pumpe geöffnet ist. Aus Sicherheitsgründen ist ein Pumpenbetrieb bei geöffneter Frontscheibe nicht möglich.

Über die Folientastatur werden die variablen Pumpdaten dann eingegeben.

Mit den Tasten (heating-1 an / off bei einer Heizschale; bzw. heating-2 an / off bei zwei angeschlossenen Heizschalen) werden die beiden Heizschalen ein- und ausgeschaltet. Die grünen LED zeigen an, welche Heizschalen in Betrieb sind.

Mit der Taste (Start / Stop) wird die Pumpe gestartet oder angehalten. Die gleiche Funktion führt der pneumatische Fußschalter aus.

Mit der Taste V1 wird die zeitliche Einbringungsmenge bzw. Fördermenge pro Minute (Milliliter pro Minute {ml/min}) der Pumpe eingegeben. Der kleinste Einstellwert ist z.B. 30ml/min. Der größte Einstellwert ist bevorzugt 200 ml/min.

Mit der Taste V2 wird das Einbringungsvolumen (Milliliter) eingegeben. Maximal einstellbares Einbringungsvolumen ist bei diesem Beispiel 5000 ml.

Die Tasten dienen der digitalen Veränderung obiger Eingabewerte.

Die Pumpe kann weiterhin mit dem Fußschalter gestartet oder angehalten werden. Nach Ablauf der eingestellten Anwendung ertönt aus der Pumpe ein akustisches Signal und die Pumpe bleibt stehen.

Beispiel für eine Anwendung:
→ eingestellte Fördermenge = 80 ml/min
→ ingestelltes Einbringungsvolumen = 1600 ml

Wird die Pumpe nun gestartet, so fördert die Pumpe 20 Minuten lang pro Minute 80 ml. (1600 (ml) / 80 (ml) = 20 min).

Die Pumpenfunktion kann während der Anwendung mit dem Fußschalter oder mit der Start / Stop-Taste unterbrochen werden. In diesem Fall werden die eingestellten Werte nur angehalten (Unterbrechung). Wird der Pumpvorgang jetzt wieder gestartet, so wird der eingestellte Vorgang weiter abgearbeitet. Ist der Pumpvorgang abgeschlossen, so ertönt ein akustisches Signal und die Pumpe bleibt stehen.

Die Löschung der eingestellten Pumpendaten während der Anwendung oder während der Unterbrechung ist nur durch eine komplette Abschaltung der Pumpe über den Netzschalter möglich.

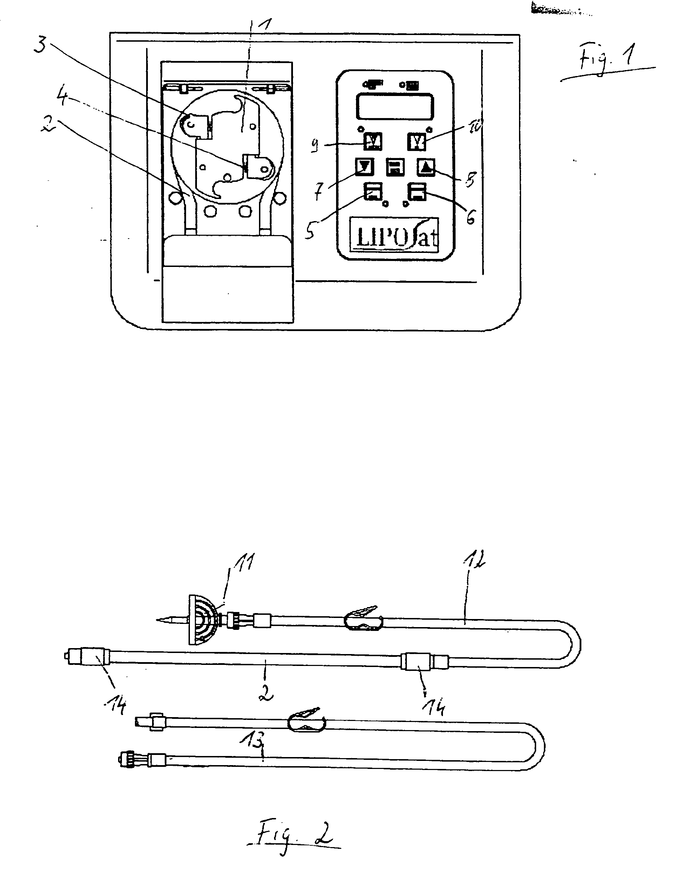

In Figur 1 ist eine Draufsicht auf die Tumeszenzpumpe dargestellt. Auf der linken Seite der Abbildung ist die Schlauchpumpe 1 dargestellt. Bestandteile dieser Schlauchpumpe 1 sind der Pumpschlauch 2, die Antriebsrolle 3 und die Feder 4. Auf der rechten Seite dieser Figur sind die Schalter ein/aus für die Heizungen 5 und 6, der Schalter für digitale Veränderung des Vorgabewertes der Fördermenge pro Zeiteinheit 7, der Schalter für digitale Veränderung des Vorgabewertes der Gesamtfördermenge 8, der Schalter für die Einstellung des Wertes der Fördermenge pro Zeiteinheit 9 und der Schalter für die Einstellung des Wertes der Gesamtfördermenge 10 dargestellt.

In Figur 2 ist das Schlauchsystem abgebildet. Es besteht aus dem Pumpschlauch 2, dem Einstechdorn 11, dem Ansaugschlauch 12, dem Verbindungsschlauch 13 und dem Adaptionsstück 14.

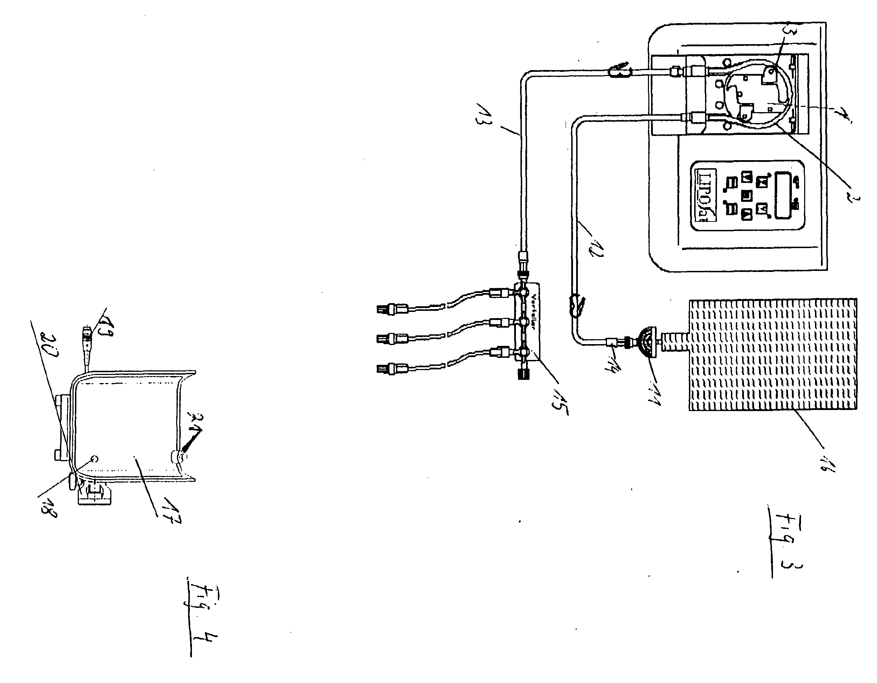

In Figur 3 ist eine Anordnung mit Heizschale und eingelegtem Kochsalzlösungsbeutel 16 gezeigt; die Heizschale ist dabei bildlich nicht dargestellt, da sie sich perspektivisch hinter dem Kochsalzlösungsbeutel 16 befindet. Weiter ist auf dieser Abbildung die Schlauchpumpe 1 mit eingelegtem Pumpschlauch 2 und den Antriebsrollen 3 zu sehen. Der Kochsalzlösungsbeutel 16 ist über den Einstechdorn 11, das Adaptionsstück 14 und den Ansaugschlauch 12 mit der Schlauchpumpe 1 verbunden. Diese wiederum ist mittels des Verbindungsschlauches 13 mit einem Verteiler 15 verbunden.

In Figur 4 ist eine Heizschale 17 mit dem Temperatursensor 18, dem Anschlussstecker für die Heizung 19, einer Befestigungsschelle für den Adapter des Chirurgieabsaugers 20 und einer Halterung 21 für die Kochsalzlösungs-beutel 16 dargestellt.

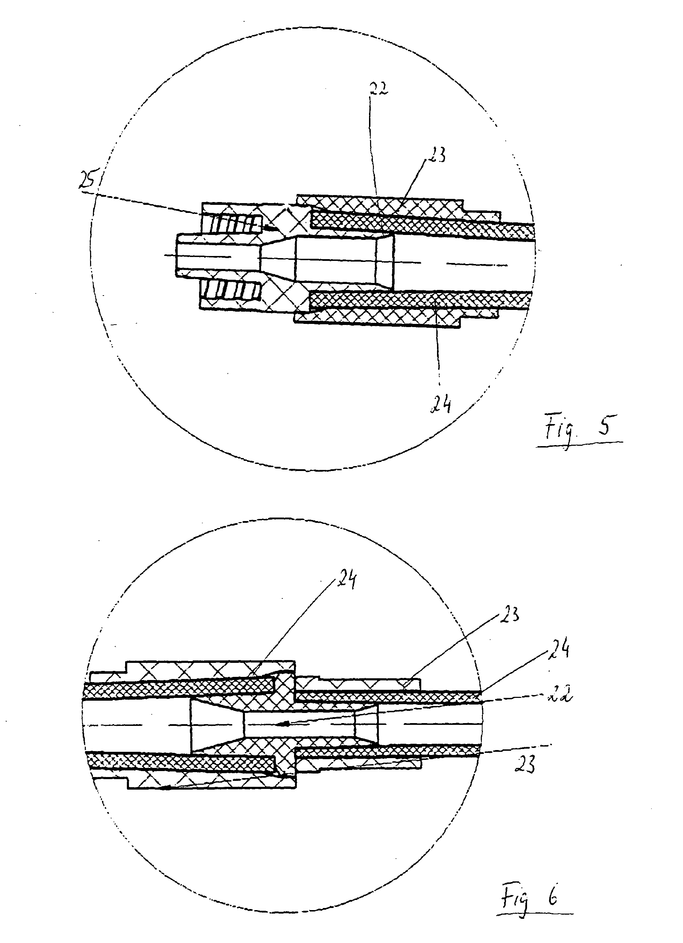

In den Figuren 5 und 6 ist die Schlauchverbindung mit dem Schlauchanschluss 22, der Hülse 23, dem Schlauchteil 24 zu sehen. In Figur 5 ist dabei die Verbindung des Schlauchteils 24 mit einer Luer-Verbindung 25 und in Abbildung 6 die Verbindung zweier Schlauchteile 24 miteinander abgebildet.

## Patentansprüche

1. Tumeszenzpumpe zum sterilen Fördern erwärmter Tumeszenzlösung in das Gewebe eines Patienten, insbesondere zur Regionalanästhesie des intrakutanen und subkutanen Gewebes mit
einer Schlauch-Rollenpumpe (1) mit Antriebsrollen (3),
einer Heizeinrichtung, die einen Wärmesensor (18), der die Temperatur der Tumeszenzlösung erfasst, und wenigstens eine Heizschale (17) aufweist, die die aus dem oder den Tumeszenzlösungs-Vorratsbeuteln (16) austretende Tumeszenzlösung auf eine einstellbare Temperatur erwärmt,
einem sterilen Einmal-Schlauchset (2, 12, 13),
einer Steuereinheit,
einer Ausgabeeinheit, die alle variablen Pumpdaten während des Pumpvorgangs erfasst, speichert und während oder nach Ablauf der Anwendung ausgibt,
**dadurch gekennzeichnet,**
**dass** die Steuereinheit so eingerichtet ist, dass sie die Einstellung
a) der Gesamtmenge der einzubringenden vorgewärmten Tumeszenzlösung, und
b) der Flussrate (zeitliche Einbringungsmenge) erlaubt, und dass sie die Schlauch-Rollenpumpe entsprechend dieser Vorgabewerte steuert sowie den Einbringvorgang bei Erreichen der eingestellten Gesamtmenge beendet,
und **dass** die Antriebsrollen (3) der Pumpe (1) federgelagert (4) sind, wobei die Federn auf Druck derart belastbar sind, dass sie bei einem vorgewählten Druck nachgeben, so dass die Antriebsrollen den Pumpschlauch (2) nicht mehr zusammendrücken können und keine Förderung von Flüssigkeit erfolgen kann.

2. Tumeszenzpumpe nach Anspruch 1, **dadurch gekennzeichnet, dass** die Steuereinheit die Pumpe derart steuert, dass während einer Umdrehung der Antriebsrollen der Pumpe die geförderte Menge konstant gehalten wird.

3. Tumeszenzpumpe nach Anspruch 1 oder 2, **dadurch gekennzeichnet, dass** der Schlauchset wenigstens drei Bestandteile, nämlich einen Ansaugschlauch (12), einen Pumpschlauch (2) und einen Verbindungsschlauch (13) aufweist.

4. Tumeszenzpumpe nach Anspruch 3, **dadurch gekennzeichnet, dass** der Pumpschlauch (2) aus einem Kunststoffmaterial mit einer Festigkeit von 55 Shore, +/- 5 Shore besteht.

5. Tumeszenzpumpe nach Anspruch 4, **dadurch gekennzeichnet, dass** die Verbindung des Ansaugschlauches bzw. des Verbindungsschlauches zur Tumeszenzpumpe mittels einer Luer-Verbindung (25) realisiert ist, wobei diese zur Aufnahme des jeweiligen Schlauches einen konisch ausgebildeten Ansatz aufweist, auf welchen der jeweilige Schlauch aufgezogen und verklebt ist, und dass über den Schlauch außen eine Hülse (23) angebracht und verklebt ist.

6. Tumeszenzpumpe nach einem der Ansprüche 1 bis 5, **dadurch gekennzeichnet, dass** die Pumpe mittels eines Fußschalters bedienbar ist.

7. Tumeszenzpumpe nach einem der Ansprüche 1 bis 6, **dadurch gekennzeichnet, dass** an der Pumpe eine Befestigungseinrichtung für einen Chirurgieabsauger vorhanden ist, über die der Chirurgieabsauger mit der Tumeszenzpumpe lösbar verbunden ist.

8. Tumeszenzpumpe nach Anspruch 7, **dadurch gekennzeichnet, dass** die Pumpe und der Absauger über einen Doppel-Fußschalters bedienbar sind.

## Claims

1. A tumescence pump for sterile delivery of heated tumescence solution into the tissue of a patient, especially for regional anesthesia of the intracutaneous and subcutaneous tissue, comprising
a roller-hose pump (1) with drive rollers (3),
a heating device, which has a heat sensor (18) for detecting the temperature of the tumescence solution, and at least one heating dish (17), which heats the tumescence solution coming out of the tumescence solution storage bag(s) (16) to an adjustable temperature,
a sterile disposable hose set (2, 12, 13),
a control unit,
an output unit which detects all variable pump data during pump operation, saves and outputs the data during or after use,
**characterized in that**
the control unit is set up so that it allows the adjustment of
a) the total quantity of preheated tumescence solution to be introduced, and
b) the flow rate (quantity to be introduced over time), controlling the roller-hose pump according to these specified values and terminating the introduction operation on reaching the preset total quantity,
and the drive rollers (3) of the pump (1) are springloaded (4), such that the springs can be put under load under load so that they yield at a preselected pressure and so that the drive rollers can no longer compress the pump hose (2) and there can be no delivery of fluid.

2. The tumescence pump according to Claim 1,
**characterized in that**
the control unit controls the pump such that the quantity delivered is kept constant during one revolution of the drive rollers of the pump.

3. The tumescence pump according to Claim 1 or 2,
**characterized in that**
the hose set has at least three components, namely an intake hose (12), a pump hose (2) and a connecting hose (13).

4. The tumescence pump according to Claim 3,
**characterized in that**
the pump hose (12) is made of a plastic material having a strength of 55 Shore V 5 Shore.

5. The tumescence pump according to Claim 4,
**characterized in that**
the connection of the intake hose and/or the connecting hose to the tumescence pump is accomplished via a Luer connection (25), whereby this accommodation of the respective hose has a conical nipple to which the respective hose is attached and bonded and a sleeve (23) is attached to the hose from the outside and bonded in place.

6. The tumescence pump according to any one of Claims 1 through 5,
**characterized in that**
the pump can be operated by means of a foot switch.

7. The tumescence pump according to any one of Claims 1 through 6,
**characterized in that**
a fastening device for a surgical suction apparatus is provided on the pump by which the surgical suction device is detachably connected to the tumescence pump.

8. The tumescence pump according to Claim 7,
**characterized in that**
the pump and the suction device can be operated via a double foot switch.

## Revendications

1. Pompe à tumescence pour le transport stérile d'une solution tumescente dans le tissu d'un patient, notamment pour l'anesthésie locale du tissu intracutané et sous-cutané, comportant
une pompe de Bakey tubulaire (1) équipée de rouleaux de propulsion (3),
un dispositif de chauffage qui comporte un capteur thermique (18) enregistrant la température de la solution tumescente et au moins une coque chauffante qui chauffe la solution tumescente sortant du ou des sacs de réserve de solution tumescente (16) à une température réglable,
un ensemble de tuyaux stériles à usage unique (2, 12, 13),
une unité de commande,
une unité d'édition qui enregistre, sauvegarde et édite pendant ou après la fin de l'application toutes les données variables sur la pompe pendant le processus de pompage,
**caractérisée en ce que**
l'unité de commande est aménagée de manière à permettre le réglage
a) de la quantité totale de solution tumescente préchauffée à injecter et
b) du débit d'écoulement (quantité introduite dans le temps) et à commander la pompe de Bakey tubulaire en fonction de ces valeurs prescrites ainsi qu'à terminer l'opération d'injection une fois que la quantité totale réglée est atteinte,
et **en ce que** les rouleaux de propulsion (3) de la pompe (1) ont un appui à ressorts (4), les ressorts pouvant être sollicités à une pression présélectionnée, de sorte que les rouleaux de propulsion ne peuvent plus comprimer le tuyau de la pompe (2) et qu'il ne peut y avoir aucun transport de liquide.

2. Pompe à tumescence selon la revendication 1, **caractérisée en ce que** l'unité de commande contrôle la pompe de manière à ce que, pendant une rotation des rouleaux de propulsion de la pompe, la quantité transportée reste constante.

3. Pompe à tumescence selon la revendication 1 ou 2, **caractérisée en ce que** l'ensemble de tuyaux comporte au moins trois composantes, à savoir un tuyau d'aspiration (12), un tuyau de pompe (2) et un tuyau de liaison (13).

4. Pompe à tumescence selon la revendication 3, **caractérisée en ce que** le tuyau de la pompe (2) est composé d'un matériau de matière plastique ayant une résistance de 56 Shore, +/- 5 Shore.

5. Pompe à tumescence selon la revendication 4, **caractérisée en ce que** la liaison du tuyau d'aspiration ou du tuyau de liaison et de la pompe à tumescence est établie au moyen d'une connexion Luer (25), celle-ci présentant, pour recevoir le tuyau respectif, une pièce ajoutée de conformation conique sur laquelle le tuyau respectif est tiré et collé, et qu'un manchon (23) est installé et collé sur l'extérieur du tuyau.

6. Pompe à tumescence selon une des revendications 1 à 5, **caractérisée en ce que** la pompe peut être actionnée au moyen d'un interrupteur à pédale.

7. Pompe à tumescence selon une des revendications 1 à 6, **caractérisée en ce qu'**il y a sur la pompe un dispositif de fixation pour un aspirateur chirurgical, qui relie de manière détachable l'aspirateur chirurgical à la pompe à tumescence.

8. Pompe à tumescence selon la revendication 7, **caractérisée en ce que** la pompe et l'aspirateur peuvent être actionnés au moyen d'un double interrupteur à pédale.
